# EUROPEAN PATENT APPLICATION

(11) **EP 4 843 609 A2**
(43) Date of publication of application: **25.11.2026**
(21) Application number: 26200892.3
(22) Date of filing: 07.11.2023
(51) Int. Cl.: A61N 1/36

(54) **SYSTEM FOR MICTURITION CONTROL IN A MAMMAL WITH BLADDER DISFUNCTION**

(30) Priority: 07.11.2022 EP 22205945
(62) Document number(s) of the earlier application(s) in accordance with Art. 76 EPC: 23801452.6 / 4 615 562
(71) Applicant: Ecole Polytechnique Fédérale de Lausanne, 1015 Lausanne (CH); UTI Limited Partnership, Calgary, AB T2L 1Y8 (CA)
(72) Inventor: COURTINE, Grègoire, 1003 Lausanne (CH); BLOCH, Jocelyne, 1094 PAUDEX (CH); SQUAIR, Jordan, 1003 Lausanne (CH); PHILLIPS, Aaron, Calgary, Alberta T2P0X2 (CA); MAHE, Loïs, 1205 Geneve (CH)
(74) Representative: K&L Gates LLP

(57) **Abstract**

The present invention relates to a neuromodulation/neurostimulation system (10) for stimulating at least one neuronal circuitry responsible for micturition control in a mammal with bladder disfunction, especially for bladder relaxation and/or for bladder voiding, said system (10) comprising: - at least one control unit (12), and - at least one stimulation unit (14), configured and arranged to provide electrical stimulation to the spinal cord of said mammal. Further, the present invention relates to the use of the neuromodulation/neurostimulation system (10).

## Description

The present invention belongs to the technical field of spinal cord stimulation for rehabilitation of an autonomic function in a mammal, in particular a human.

More specifically, the present invention refers to a neuromodulation/neurostimulation system for micturition control in a mammal, in particular a human, having bladder disfunction after spinal cord injury (SCI) and/or other neurodegenerative and/or neurological disorders.

The spinal cord is an integral part of the central nervous system (CNS). SCI may result not only in motor and sensory deficits, but also in autonomic dysfunctions.

Specifically, SCI may result in disconnection of some, most, or all descending sympathetic, parasympathetic, and/or somatic pathways that carry signals responsible for regulating, e.g., bladder function, gut function, arterial blood pressure and/or heart rate.

In particular, the main functions of the lower urinary tract that may be compromised after SCI are the ability to store and to expel urine in a coordinated, controlled manner (de Groat et al. (1998) Behav. Brain Res. 92: 127-140; Shefchyk (2002) Progr. Brain Res. 137: 71-82).

Bladder disfunctions may also follow as a consequence of neurological disorders such as a stroke, multiple sclerosis, autonomic failure, autonomic neuropathy, other neurodegenerative disorders, or cancer of neurological tissue, which impair operation of descending sympathetic, parasympathetic, and/or somatic pathways that normally facilitate control of autonomic functions.

Experimental evidence on normal adult rats has shown that storage of urine is dependent on the inhibition of parasympathetic action on the detrusor (i.e., smooth bladder muscle) and on the sympathetic tonic activation of the internal urethral sphincter for outflow resistance. During micturition, efficient voiding is dependent on synchronous activation of the detrusor muscle for contraction, relaxation of the internal urethral sphincter, and bursting activity of the striated external urethral sphincter (EUS) for enhanced urine flow (Maggi et al. (1986) J. Pharmacol. Meth., 15: 157-167; Kruse et al. (1993) Am J. Physiol. -Regul. Integrative and Comp. Physiol., 264: 1157-1163).

In humans, conscious control of the initiation of these largely autonomic functions involves a complex interaction between the cerebral cortex, the pontine micturition center, the sympathetic and parasympathetic nervous systems, and somatic motorneurons in the lumbar and sacral spinal cord.

This interaction simultaneously activates stereotypical postural adjustments that are species as well as gender unique.

Bladder dysfunction following SCI and/or other neurodegenerative and/or neurological disorders is a potentially life-threatening condition that leads to incontinence, urinary infections and potentially ensuing renal insufficiency or even cancer. A dysregulated bladder has a significant impact on everyday life of a subject, and further requires a strict medical follow up. Thus, management of bladder disfunction results in a burdensome task both for the patient and the health care system. Accordingly, bladder function is consistently ranked among the top health priorities.

Detrusor hyperreflexia and urethral sphincter dyssynergia are hallmarks clinical signs in subjects affected by SCI above the conus, and are due to aberrant plasticity of the neuronal circuitry below the lesion. Consequently, physiological stimuli such as minor bladder wall distention or irritation can become a source of reactive and deleterious bladder contraction.

Detrusor hyperreflexia and urethral sphincter dyssynergia may also be the consequence of other neurodegenerative and/or neurological disorders.

Over the past several decades, multiple techniques have been used to induce micturition after SCI and/or other neurodegenerative and/or neurological disorders, including stimulation of the bladder wall, the pelvic nerve, and/or the sacral nerve. Directly stimulating the bladder wall induces local contractions, but high currents or a large number of electrodes are needed to induce a more widespread contraction to achieve sufficient bladder emptying. Pelvic nerve stimulation has been shown to contract the bladder wall but, as the pelvic nerve does not innervate the EUS, minimal effect was seen on the EUS resulting in a low voiding efficiency (Holmquist and Tord (1968) Scand. J. Urol. Nephrol. 2: 129-135).

An enhanced result in terms of bladder voiding could be achieved by cutting the pudendal nerve. Nevertheless, this results in a largely irreversible procedure, that also eliminates sensation from the external genitalia of both sexes and the skin around the anus and perineum, as well as the motor supply to various pelvic muscles, including the external urethral sphincter and the external anal sphincter. Sacral nerve stimulation seemed to offer the best results, but requires complicated surgical procedures and a serious risk of permanent damage via the intradural approach (Rijkhoff et al. (1997) J. Urol., 157: 1504-1508).

Partial solutions to the aforementioned drawbacks of the prior art have been provided in more recent times.

WO2018148844A1 discloses an apparatus for automated control of a dysregulated autonomic function, e.g., bladder control, in a subject affected by SCI or other neurological conditions such as multiple sclerosis, autonomic failure, autonomic neuropathy, or cancer of the neurological tissue. The apparatus includes a monitor for measuring a parameter, e.g. bladder volume or bladder pressure, of an autonomic function of the subject. The apparatus further includes a control circuit configured to receive and analyze information (e.g., relating to bladder volume or bladder pressure) from the monitor and determine whether a measured parameter value is acceptable, too high or too low. Then, in response to this determination, the control circuit controls a stimulation device to apply stimulation to the subject.

US2007027495A1 discloses an implantable bladder sensor attachable to an exterior surface of the urinary bladder of a subject to sense a bladder condition or activity for urinary incontinence or an inability to control urinary function. The sensor includes a strain gauge that detects mechanical deformation of the bladder. Mechanical deformation may be indicative of a gradual filling of the bladder, or an instantaneous contraction indicating an imminent urine voiding event. Wireless telemetry circuitry within the sensor transmits information to implanted electrical stimulator that delivers electrical stimulation for alleviating urinary incontinence, or to an external programmer that controls the implanted stimulator.

Nevertheless, said known systems and methods for micturition are often limited by the fact that the mechanisms underlying detrusor hyperreflexia and urethral sphincter dyssynergia are not completely understood.

Thus, there is a strongly-felt need for improvements in treatment of urodynamic dysfunctions after SCI and/or other neurodegenerative and/or neurological disorders.

It is therefore an object of the present invention to provide a neuromodulation/neurostimulation system that enables precise control of an urological function in a mammal, in particular a human, after SCI and/or other neurodegenerative and/or neurological disorders.

In particular, there is the need for a rationally focused solution designed to specifically target sympathetic nervous system structures responsible for urodynamic functions, and to define a stimulation paradigm enabling precise biomimetic control over urodynamic functions of a subject after SCI and/or other neurodegenerative and/or neurological disorders.

This object is achieved by the provision of a neuromodulation/neurostimulation system according to claim 1.

The present invention provides a neuromodulation/neurostimulation system for stimulating at least one neuronal circuitry responsible for micturition control in a mammal having bladder dysfunction, especially for bladder relaxation and/or for bladder voiding.

In particular, said mammal may be a human affected by SCI, other neurological disorders such as a stroke, multiple sclerosis, autonomic failure, autonomic neuropathy, other neurodegenerative diseases, and/or cancer of neurological tissue, which impair operation of descending sympathetic, parasympathetic, and/or somatic pathways that normally facilitate control of autonomic functions.

The system comprises at least one control unit.

In one embodiment, the system may comprise a single control unit.

The system further comprises at least one stimulation unit.

The at least one stimulation unit is configured and arranged to provide electrical stimulation to the spinal cord of said mammal, in particular a human.

The stimulation unit may include one or more implantable electrode arrays.

Advantageously, the at least one control unit is configured and arranged to control the at least one stimulation unit to provide low-frequency electrical stimulation to the last lumbar spinal segment and/or the first two sacral segments of the spinal cord of said mammal, especially at level L5-S1 of the spinal cord, to stimulate autonomic neurons responsible for detrusor contractions.

Additionally or alternatively, the at least one control unit is configured and arranged to control the at least one stimulation unit to provide high-frequency electrical stimulation at sacral level, especially to the first three sacral segments of the spinal cord of said mammal, to modulate sacral motoneurons responsible for external urethral sphincter relaxation.

Advantageously, the low-frequency electrical stimulation to the last lumbar spinal segment and/or the first two sacral segments, especially at level L5-S1 of the spinal cord, and high-frequency electrical stimulation at sacral level, especially to the first three sacral segments of the spinal cord of said mammal, are delivered simultaneously.

The invention is based on the basic idea that, by providing a neuromodulation/neurostimulation system that is capable of specifically targeting and stimulating neuronal circuitry responsible for micturition control, it is possible to obtain precise control over urodynamic functions in a subject having bladder disfunctions after SCI and/or other neurodegenerative and/or neurological disorders.

Preferably, this may be achieved by the provision of an improved neuromodulation/neurostimulation system which is configured and arranged to simultaneously deliver low-frequency electrical stimulation to the last lumbar spinal segment and/or the first sacral segment of the spinal cord of said mammal, especially at level L5-S1 of the spinal cord, to stimulate autonomic neurons responsible for detrusor contraction, and high-frequency electrical stimulation at sacral level, especially to the first three sacral segments of the spinal cord, to modulate sacral motoneurons responsible for external urethral sphincter relaxation, for synergic activity.

This may also be achieved by the provision of an improved neuromodulation/neurostimulation system which is configured and arranged to activate ascending pathways that cause a positive feedback loop including neurons in the pontine micturition centre to restore the natural activation of the micturition reflex in the lumbosacral spinal cord.

Thus, it is possible to obtain a precise and synergic control of the detrusor and the external urethral sphincter activity after SCI and/or other neurodegenerative and/or neurological disorders.

The at least one control unit is configured and arranged to control the at least one stimulation unit to provide electrical stimulation over a predefined period of time.

The predefined period of time may be specifically tuned based on specific conditions of a subject.

In particular, the predefined period of time may range between seconds and days.

The neuromodulation/neurostimulation system may further include a sensor unit.

In particular, the sensor unit may be configured and arranged to detect one or more physiological values of said mammal, in particular a human, among:
- intra-bladder pressure;
- detrusor contraction, and/or
- external urethral sphincter pressure.

Advantageously, the sensor unit may include one or more biocompatible implantable sensors.

The sensor unit may be configured and arranged to transmit the one or more detected physiological values to the control unit.

The control unit may be configured and arranged to operate the stimulation unit based on the one or more detected physiological values from the sensor unit.

Accordingly, the neuromodulation/neurostimulation system operates as a closed-loop system.

The low-frequency electrical stimulation for detrusor contraction may be delivered at a frequency between 10 and 700 Hz.

Preferably, the low-frequency electrical stimulation for detrusor contraction may be delivered at a frequency between 10 and 500 Hz.

More preferably, the low-frequency electrical stimulation for detrusor contraction may be delivered at a frequency between 10 and 200 Hz.

Advantageously, the low-frequency electrical stimulation for detrusor contraction may have an amplitude between 0 and 50 mA.

Advantageously, the low-frequency electrical stimulation for detrusor contraction may have a pulse width between 1-50 µs.

Preferably, the low-frequency electrical stimulation for detrusor contraction may be delivered at a frequency between 10 and 500 Hz with amplitude between 0 and 50 mA and pulse width between 1 and 50 µs.

More preferably, the low-frequency electrical stimulation for detrusor contraction may be delivered at a frequency between 10 and 200 Hz with amplitude between 0 and 50 mA and pulse width between 1 and 50 µs.

As an alternative, low-frequency electrical stimulation for detrusor contraction may be delivered according to one or more burst stimulation patterns.

Preferably, the one or more burst stimulation patterns may include 0 to 20 pulses per burst.

In particular, the one or more burst stimulation patterns may have a carrier frequency between 10 and 700 Hz.

Preferably, the one or more burst stimulation patterns may have a carrier frequency between 0 and 500 Hz.

More preferably, the one or more burst stimulation patterns may have a carrier frequency between 0 and 200 Hz.

Advantageously, the one or more burst stimulation patterns may have a burst frequency between 100 and 5000 Hz.

Advantageously, the one or more burst stimulation patterns may have an amplitude between 0 and 50 mA.

Preferably, the low-frequency electrical stimulation for detrusor contraction is delivered according to one or more burst stimulation patterns including 0 to 20 pulses per burst, with carrier frequency between 10 and 500 Hz, burst frequency between 100 and 5000 Hz, and amplitude between 0 and 50 mA.

More preferably, the low-frequency electrical stimulation for detrusor contraction is delivered according to one or more burst stimulation patterns including 0 to 20 pulses per burst, with carrier frequency between 10 and 200 Hz, burst frequency between 100 and 5000 Hz, and amplitude between 0 and 50 mA.

The high-frequency electrical stimulation for external urethral sphincter relaxation may be delivered at a frequency between 100 and 20000 Hz.

Preferably, the high-frequency electrical stimulation for external urethral sphincter relaxation may be delivered at a frequency of 10000 Hz.

Advantageously, the high-frequency electrical stimulation for external urethral sphincter relaxation may be delivered according to one or more burst stimulation patterns.

In particular, the one or more burst stimulation patterns may include 0 to 20 pulses per burst.

Advantageously, the one or more burst stimulation patterns may have a pulse width between 1 and 500 µs.

Advantageously, the one or more burst stimulation patterns may have an amplitude between 0 and 50 mA.

Preferably, the high-frequency electrical stimulation for external urethral sphincter relaxation is delivered according to one or more burst stimulation patterns including 0 to 20 pulses per burst, with pulse width between 1 and 500 µs and amplitude between 0 and 50 mA.

The present invention further relates to the use of a neuromodulation/neurostimulation system as described above in the treatment of a bladder disfunction in a mammal, in particular a human, after SCI and/or other neurodegenerative and/or neurological disorders.

The neuromodulation/neurostimulation system of the invention may be used in a method for stimulating neuronal circuitry responsible for micturition control in a mammal with bladder disfunction, especially for bladder relaxation and/or for bladder voiding.

In particular, said mammal may be a human affected by SCI, other neurological disorders such as a stroke, multiple sclerosis, autonomic failure, autonomic neuropathy, other neurodegenerative disorders, or cancer of neurological tissue, which impair operation of descending sympathetic pathways that normally facilitate control of autonomic functions.

The method includes positioning at least one stimulation unit on the body of a mammal, in particular a human, to deliver stimulation to the spinal cord of said mammal.

Advantageously, the method further includes delivering low-frequency electrical stimulation to the last spinal segment and/or the first two sacral segments of the spinal cord of said mammal, especially at level L5-S1of the spinal cord, to stimulate autonomic neurons responsible for detrusor contraction.

Additionally or alternatively, the method further includes delivering high-frequency electrical stimulation at sacral level, especially to the first three sacral segments of the spinal cord of said mammal, to modulate sacral motoneurons responsible for external urethral sphincter relaxation.

Preferably, the low-frequency electrical stimulation to the last lumbar spinal segment and/or the first two sacral segments of the spinal cord of said mammal, especially at level L5-S1 of the spinal cord, and the high-frequency electrical stimulation at sacral level, especially to the first three sacral segments of the spinal cord of said mammal, are delivered simultaneously.

The method may include providing electrical stimulation over a predefined period of time.

The predefined period of time may be specifically tuned based on specific conditions of a subject.

In particular, the predefined period of time may range between seconds and days.

The method may further include:
- detecting one or more physiological values of said mammal, in particular a human, through a sensor unit;
- transmitting the one or more detected values to the to a control unit, and
- operating the stimulation unit based on the one or more detected values from the sensor unit.

The sensor unit may include one or more biocompatible implantable sensors.

In particular, the physiological values may include one or more among:
- intra-bladder pressure;
- detrusor contraction, and/or
- external urethral sphincter pressure.

The method may include delivering the low-frequency electrical stimulation for detrusor contraction at a frequency between 10 and 700 Hz.

Preferably, the method may include delivering the low-frequency electrical stimulation for detrusor contraction at a frequency between 10 and 500 Hz.

More preferably, the method may include delivering the low-frequency electrical stimulation for detrusor contraction at a frequency between 10 and 200 Hz.

Advantageously, the method may include delivering the low-frequency electrical stimulation for detrusor contraction with an amplitude between 0 and 50 mA.

Advantageously, the method may include delivering the low-frequency electrical stimulation for detrusor contraction with a pulse width between 1 and 50 µs.

Preferably, the method may include delivering the low-frequency electrical stimulation for detrusor contraction at a frequency between 10 and 500 Hz with amplitude between 0 and 50 mA and pulse width between 1 and 50 µs.

More preferably, the method may include delivering the low-frequency electrical stimulation for detrusor contraction at a frequency between 10 and 200 Hz with amplitude between 0 and 50 mA and pulse width between 1 and 50 µs.

As an alternative, the method may include delivering the low-frequency electrical stimulation for detrusor contraction according to one or more burst stimulation patterns.

Preferably, the one or more burst stimulation patterns may include 0 to 20 pulses per burst.

In particular, the one or more burst stimulation patterns may have a carrier frequency between 10 and 700 Hz.

Preferably, the one or more burst stimulation patterns may have a carrier frequency between 0 to 500 Hz.

More preferably the one or more burst stimulation patterns may have a carrier frequency between 0 to 200 Hz.

Advantageously, the one or more burst stimulation patterns may have burst frequency between 100 and 5000 Hz.

Advantageously, the one or more burst stimulation patterns may have an amplitude between 0 and 50 mA.

Preferably, the method may include delivering the low-frequency electrical stimulation for detrusor contraction according to one or more burst stimulation patterns including 0 to 20 pulses per burst, with carrier frequency between 10 and 500 Hz, burst frequency between 100 and 5000 Hz, and amplitude between 0 and 50 mA.

More preferably, the method may include delivering the low-frequency electrical stimulation for detrusor contraction according to one or more burst stimulation patterns including 0 to 20 pulses per burst, with carrier frequency between 10 and 200 Hz, burst frequency between 100 and 5000 Hz, and amplitude between 0 and 50 mA.

The method may include delivering the high-frequency electrical stimulation for external urethral sphincter relaxation at a frequency between 100 and 20000 Hz.

Preferably, the method may include delivering the high-frequency electrical stimulation for external urethral sphincter relaxation at a frequency of 10000 Hz.

Advantageously, the method may include delivering the high-frequency electrical stimulation for external urethral sphincter relaxation according to one or more burst stimulation patterns.

Preferably, the one or more burst stimulation patterns may include 0 to 20 pulses per burst.

Advantageously, the one or more burst stimulation patterns may have a pulse width between 1 and 500 µs.

Advantageously, the one or more burst stimulation patterns may have an amplitude between 0 and 50 mA.

Preferably, the method may include delivering the high-frequency electrical stimulation for external urethral sphincter relaxation according to one or more burst stimulation patterns including 0 to 20 pulses per burst, with pulse width between 1 and 500 µs and amplitude between 0 and 50 mA.

Further details and advantages of the present invention shall now be disclosed in connection with the drawings, where:
**Fig. 1** is a diagram showing a schematic overview of a neuromodulation/neurostimulation system according to an embodiment of the present invention;
**Fig. 2** is a diagram showing an exemplary setup for a neuromodulation/neurostimulation system for bladder relaxation;
**Fig. 3** is a diagram showing an exemplary setup for a neuromodulation/neurostimulation system for bladder relaxation that is similar to the one of **Fig. 2****,** but where the system is further equipped with one or more control sensors;
**Fig. 4** is a diagram showing an exemplary setup for a neuromodulation/neurostimulation system for bladder relaxation that is similar to the one of **Fig. 2****,** but where the system is further equipped with one or more bladder sensors;
**Fig. 5** is a diagram showing an exemplary setup for a neuromodulation/neurostimulation system for bladder voiding;
**Fig. 6** is a diagram showing an exemplary setup for a neuromodulation/neurostimulation system for bladder voiding that is similar to the one of **Fig. 5****,** but where the system is further equipped with one or more control sensors;
**Fig. 7** is a diagram showing an exemplary setup for a neuromodulation/neurostimulation system for bladder voiding that is similar to the one of **Fig. 5****,** but where the system is further equipped with one or more bladder sensors and/or one or more urethral sphincter sensors.
**Fig. 8** is a diagram showing functional mapping results in rats after stimulating each segment of the spinal cord from T11 to S4, demonstrating intra-bladder pressure optimal response at spinal cord segments L5-L6;
**Fig. 9** is a diagram showing functional mapping results in rats after stimulating each segment of the spinal cord from T11 to S4, demonstrating external urethral sphincter optimal response at spinal cord segments S1-S2;
**Fig. 10** is a diagram showing results of retrograde tracing from the bladder wall by counting labeled spinal neurons for each segment of the spinal cord from T9 to S4, demonstrating the highest response at spinal cord level L6 (rat model);
**Fig. 11** is a diagram showing results of retrograde tracing from the external urethral sphincter muscle by counting labeled spinal neurons for each segment of the spinal cord from T9 to S4, demonstrating the highest response at spinal cord level S1 (rat model);
**Fig. 12** is a diagram showing linear correlation between bladder functional and anatomic mappings (rat model);
**Fig. 13** is a diagram showing linear correlation between external urethral sphincter functional and anatomic mappings (rat model);
**Fig. 14** is a diagram showing external urethral sphincter silencing during bladder filling, with induced sphincter silencing using electrical stimulation at 5000 Hz at spinal cord level S1, where dotted lines denote the moment in which stimulation has been turned on (rat model);
**Fig. 15** is a diagram showing an algorithmic procedure to identify EES parameters for bladder contraction. The optimal electrode configuration was selected based on both the immediate increase in intra-bladder pressure and the absence of significant muscle contraction. The array was visualized using an intraoperative X-ray to confirm the location of the array (second panel). Lastly, intensity and frequency mapping were respectively performed in order to identify optimal parameters for bladder contraction;

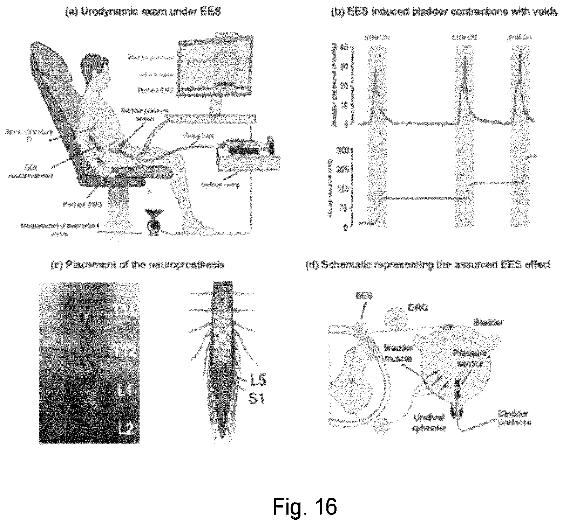

**Fig. 16** is a diagram showing preliminary data from a urodynamic exam performed under EES on a patient with spinal cord injury. Applying specific EES configuration using the bottom electrodes of the neuroprosthesis to provide low-frequency electrical stimulation to the lower lumbar/upper sacral region of the spinal cord enabled to introduce bladder contractions and to restore voiding;
**Fig. 17** is a diagram showing a specific EES configuration using high frequency through the bottom electrodes of the neuroprosthesis enable to prevent bladder contraction (overreactive bladder) on a patient with spinal cord injury;

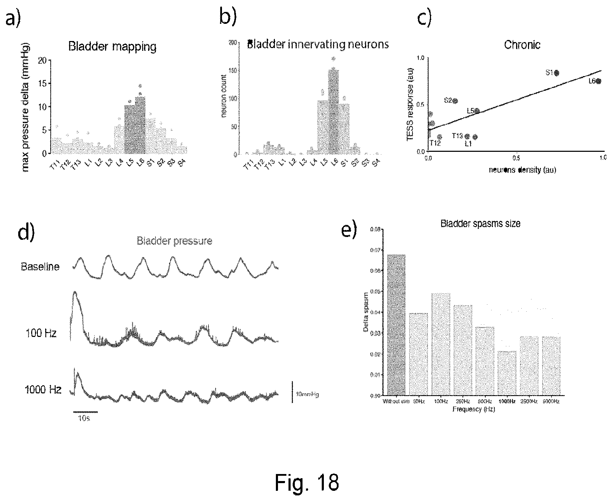

**Fig. 18** is a diagram showing how high frequency EES of the final lumbar segments relaxes the bladder. **(a)** EES of the final lumbar segments achieves the highest detrusor muscle contraction. The bar plot shows bladder pressure elevation in response to the EES of different spinal segments. **(b)** The neurons innervating the bladder are largely distributed across the final lumbar and sacral spinal cord segments. The bar plot shows the number of autonomic neurons identified in different segments of the lumbosacral spinal cord. **(c)** The distribution of autonomic neurons across the lumbosacral spinal cord correlates highly with the detrusor muscle contraction in response to EES. The scatter plot shows the relationship between the number of autonomic neurons and increase in bladder pressure caused by detrusor muscle contraction in response to single spinal segment EES across the thoracolumbosacral spinal cord. **(d)** High frequency EES reduced the amplitude of aberrant bladder contractions. The plots show bladder pressure under different EES conditions (EES off, 100 Hz, L6 EES, 1 kHz, L6 EES). (e) 1 kHz EES delivered at L6 was the most effective in relaxing the bladder. The bar plot shows the average of maximum bladder pressure during spasms for different EES conditions;

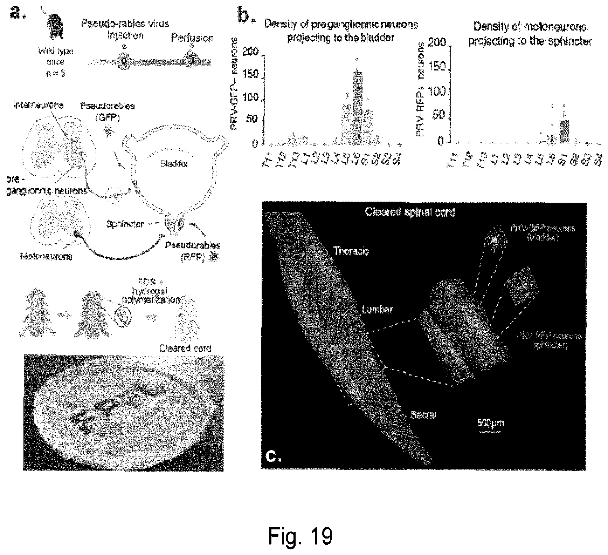

**Fig. 19** is a diagram showing how anatomical pathways involved in bladder voiding revealed by retrograde tracing viruses. **(a)** The neuronal pathways of the detrusor muscle and the urethral sphincter were traced using retrograde tracing viruses and tissue clearing technology. Five mice were injected with pseudorabies virus encoding for a fluorescent protein directly into the bladder wall and the urethral sphincter to label retrogradely the spinal neurons that project to these targets. The mice were sacrificed three days after the injection. The brain and the spinal cord were harvested, the tissue was cleared and the traced pre-ganglionic detrusor muscle neurons and the urethral sphincter neurons were counted across different spinal segments. **(b)** The density of pre-ganglionic neurons projecting to the detrusor muscle of the bladder peaked at L6 segment, and the somatic motor neurons projecting to the urethral sphincter peaked at S1 segment. The bar plots show the number of neurons across different thoracolumbosacral spinal segments. **(c)** The tri-dimensional organization of these neurons was characterized through a translucid central nervous system using tissue clearing technology. The computerized reconstruction shows the location of the traced neurons within the spinal cord;

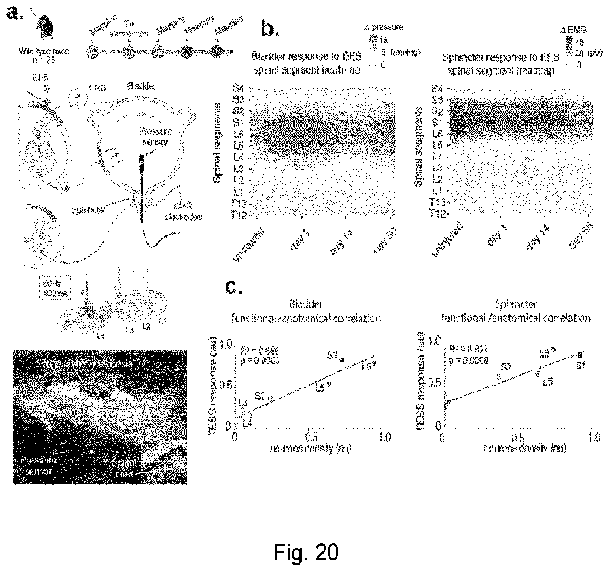

**Fig. 20** is a diagram showing how the spinal segment distribution of detrusor muscle neurons and somatic urethral sphincter neurons correlates highly with the detrusor muscle and urethral sphincter contraction, respectively, in response to single-segment EES. **(a)** The segmental distribution of detrusor muscle and urethral sphincter contraction in response to EES is stable after spinal cord injury. EES was deployed segment per segment while recording bladder pressure and electromyogram of the urethral sphincter. **(b)** The heatmaps show the bladder pressure elevation and urethral sphincter EMG evoked by single-segment EES across the thoracolumbosacral spinal cord and across four timepoints (uninjured and 1, 15 and 56 days after T9 spinal cord transection). **(c)** The maximum EES-evoked contraction of detrusor muscle and urethral sphincter was at similar levels, leading to a strong anatomical-functional correlation for both muscles. The left scatter plot shows the relationship between the number of detrusor muscle neurons and increase in bladder pressure caused by detrusor muscle contraction in response to single-segment EES across the thoracolumbosacral spinal cord. The right scatter plot shows the relationship between the number of somatic urethral sphincter neurons and urethral sphincter EMG in response to single-segment EES across the thoracolumbosacral spinal cord;

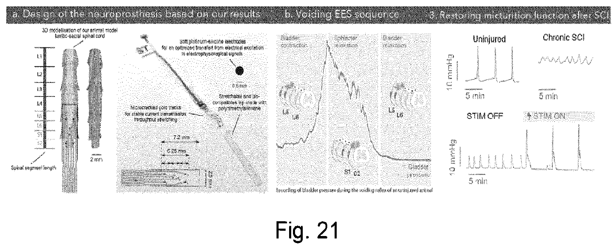

**Fig. 21** is a diagram showing how neuroprosthesis based on the biomimetic EES sequence can restore urodynamic function after SCI. **(a)** Epidural electrode arrays were developed and fabricated to target the recruitment of L5, L6, S1 and S2 segments. These arrays were based on a technology that delivers current through stretchable gold tracks covered by a highly bio-compatible material. **(b)** Biomimetic EES sequence induces bladder voiding. The sequence is composed out of three EES protocols: (i) bladder contraction, (ii) sphincter relaxation, and (iii) bladder relaxation. By combining these protocols in healthy rats implanted with our e-dura leads, it is shown that the biomimetic EES sequence results with bladder voiding. The panel shows the temporal evolution of the sequence. (c) Biomimetic EES sequence can induce regular bladder voidings to restore urodynamic function after SCI. The arrays were implanted in a T9 transection rat model of spinal cord injury. It was demonstrated that the delivery of the EES sequence results with bladder voidings;
**Fig. 22** is a diagram showing the long-term (rehabilitative) effects of stimulation. The bar graphs indicate an overall decrease in oxybutynin treatment accompanied by an increase in the volume required to trigger a bladder contraction during urodynamics, bladder capacity, and a decrease in the peak bladder contraction even when stimulation was turned off after several months of daily stimulation use.
**Fig. 23** is a diagram showing an exemplary system for recording a urinary function of a rat. **(a)** a wireless telemetry device connected to a pressure sensor inserted into the bladder and EMG electrodes implanted in the urethral sphincter. **(b)** urodynamic assessments in real time.
**Fig. 24** is a diagram showing a rehabilitation protocol.
**Fig. 25** is a diagram showing results of the urinary parameters for a group of healthy rats, injured rats, and injured rats after completion of a rehabilitation protocol.
**Fig. 26** is a diagram showing results of the micturition reflexes for a group of healthy rats, injured rats, and injured rats after the completion of a rehabilitation protocol.
**Fig. 27** is a diagram showing reinforcement of the neuronal projections in a group of healthy rats, injured rats, and injured rats after the completion of a rehabilitation protocol.

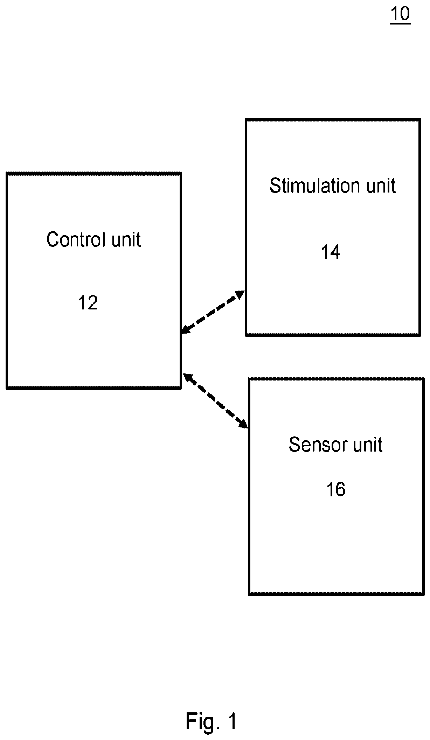

**Fig. 1** shows a schematic overview of a neuromodulation/neurostimulation system 10 according to an embodiment of the present invention.

The system 10 is configured and arranged for stimulating neuronal circuitry responsible for micturition control in a mammal having bladder disfunction, especially for bladder relaxation and/or for bladder voiding.

In particular, neuromodulation/neurostimulation paradigms have been designed that mimics natural urodynamics in a healthy subject, involving:
- relaxing the detrusor muscle;
- contracting the detrusor muscle;
- relaxing the external urethral sphincter;
- contracting the external urethral sphincter.

In particular, said mammal can be a human affected by spinal cord injury (SCI), other neurological disorders such as a stroke, multiple sclerosis, autonomic failure, autonomic neuropathy, other neurodegenerative disorder, or cancer of neurological tissue, which impair operation of descending sympathetic pathways that normally facilitate control of autonomic functions.

The system 10 includes at least one control unit 12.

In the shown embodiment, the system 10 includes a single control unit 12 (Fig. 1).

The system 10 further comprises at least one stimulation unit 14.

The at least one stimulation unit 14 is configured and arranged provide electrical stimulation to the spinal cord of said mammal, in particular a human.

Not shown is that the at least one stimulation unit 14 may include one or more implantable electrode arrays.

In the present embodiment, the control unit 12 is configured and arranged to control the at least one stimulation unit 14 to provide low-frequency electrical stimulation to the last lumbar spinal segment and/or the first two sacral segments of the spinal cord of said mammal, especially at level L5-S1 of the spinal cord, to stimulate autonomic neurons responsible for detrusor contraction.

In the present embodiment, the control unit 12 is further configured and arranged to control the at least one stimulation unit 14 to simultaneously provide high-frequency electrical stimulation at sacral level, especially to the first three sacral segments of the spinal cord of said mammal, in particular a human, to modulate sacral motoneurons responsible for external urethral sphincter relaxation.

By specifically targeting and stimulating neuronal circuitry that is responsible for micturition control, a precise control over urodynamic functions after SCI and/or other neurodegenerative and/or neurological disorders can be achieved.

In particular, by simultaneously delivering low-frequency electrical stimulation to the last lumbar spinal segment and/or the first two sacral segments of the spinal cord of said mammal, especially at level L5-S1 of the spinal cord, and high-frequency electrical stimulation at sacral level, especially to the first three sacral segments of the spinal cord of said mammal, precise and synergic control of the detrusor and the external urethral sphincter activity can be obtained.

In the present embodiment, low-frequency electrical stimulation for detrusor contraction is delivered at a frequency between 10 and 700 Hz.

Preferably, the low-frequency electrical stimulation for detrusor contraction is delivered at a frequency between 10 and 500 Hz.

More preferably, the low-frequency electrical stimulation for detrusor contraction is delivered at a frequency between 10 and 200 Hz.

Advantageously, the low-frequency electrical stimulation for detrusor contraction has an amplitude between 0 and 50 mA.

Advantageously, low-frequency electrical stimulation for detrusor contraction has a pulse width between 1-50 µs.

Based on experimental data, the inventors have defined the following low-frequency electrical stimulation parameters for detrusor muscle stimulation:
Detrusor muscle stimulation parameters - rats model

| | |
|---|---|
| Frequency: | 10-700 Hz |
| Amplitude: | 0-2000 µA |
| Pulse width: | 1-500 µs |

Detrusor muscle stimulation parameters - non-human primates model

| | |
|---|---|
| Frequency: | 10-500 Hz |
| Amplitude: | 0-50 mA |
| Pulse width: | 1-500 µs |

Detrusor muscle stimulation parameters -human model

| | |
|---|---|
| Frequency: | 10-500 Hz |
| Amplitude: | 0-50 mA |
| Pulse width: | 1-500 µs |

In humans, the low-frequency electrical stimulation for detrusor contraction is preferably delivered at a frequency between 10 and 500 Hz with amplitude between 0 and 50 mA and pulse width between 1 and 50 µs.

In humans, the low-frequency electrical stimulation for detrusor contraction is more preferably delivered at a frequency between 10 and 200 Hz with amplitude between 0 and 50 mA and pulse width between 1 and 50 µs.

As alternative, low-frequency electrical stimulation for detrusor contraction may be delivered according to one or more burst stimulation patterns.

Preferably, the one or more burst stimulation patterns include 0 to 20 pulses per burst.

In particular, the one or more burst stimulation patterns have a carrier frequency between 10 and 700 Hz.

Preferably, the one or more burst stimulation patterns have a carrier frequency between 0 to 500 Hz.

More preferably, the one or more burst stimulation patterns have a carrier frequency between 0 to 500 Hz.

Advantageously, the one or more burst stimulation patterns have burst frequency between 100 and 5000 Hz.

Advantageously, the one or more burst stimulation patterns have an amplitude between 0 and 50 mA.

Based on experimental data, the inventors have defined the following low-frequency burst stimulation parameters for detrusor muscle stimulation:
Burst stimulation parameters for detrusor muscle stimulation - rats model

| | |
|---|---|
| Pulses per burst: | 0-20 |
| Carrier frequency: | 10-700 Hz |
| Burst frequency: | 100-5000 Hz |
| Amplitude: | 0-2000 µA |

Burst stimulation parameters for detrusor muscle stimulation - non-human primates model

| | |
|---|---|
| Pulses per burst: | 0-20 |
| Carrier frequency: | 10-500 Hz |
| Burst frequency: | 100-5000 Hz |
| Amplitude: | 0-50 mA |

Burst stimulation parameters for detrusor muscle stimulation -human model

| | |
|---|---|
| Pulses per burst: | 0-20 |
| Carrier frequency: | 10-500 Hz |
| Burst frequency: | 100-5000 Hz |
| Amplitude: | 0-50 mA |

In humans, the low-frequency electrical stimulation for detrusor contraction is preferably delivered according to one or more burst stimulation patterns including 0 to 20 pulses per burst, with carrier frequency between 10 and 500 Hz, burst frequency between 100 and 5000 Hz, and amplitude between 0 and 50 mA.

In humans, the low-frequency electrical stimulation for detrusor contraction is more preferably delivered according to one or more burst stimulation patterns including 0 to 20 pulses per burst, with carrier frequency between 10 and 200 Hz, burst frequency between 100 and 5000 Hz, and amplitude between 0 and 50 mA.

In the present embodiment, the high-frequency electrical stimulation for external urethral sphincter relaxation is delivered at a frequency between 100 and 20000 Hz.

Preferably, the high-frequency electrical stimulation for external urethral sphincter relaxation is delivered at a frequency of 10000 Hz.

Advantageously, the high-frequency electrical stimulation for external urethral sphincter relaxation is delivered according to one or more burst stimulation patterns.

Preferably, the one or more burst stimulation patterns includes 0 to 20 pulses per burst.

Advantageously, the one or more burst stimulation patterns have a pulse width between 1 and 500 µs.

Advantageously, the one or more burst stimulation patterns have an amplitude between 0 and 50 mA.

Based on experimental data, the inventors have defined the following high-frequency burst stimulation parameters for detrusor and external urethral sphincter relaxation.

Burst stimulation parameters for detrusor and external urethral sphincter relaxation - rats model

| | |
|---|---|
| Frequency: | 100-20000 Hz |
| Amplitude: | 0-2000 µA |
| Pulse width: | 1-500 µs |

Burst stimulation parameters for detrusor and external urethral sphincter relaxation - non-human primates model

| | |
|---|---|
| Frequency: | 100-20000 Hz |
| Amplitude: | 0-50 mA |
| Pulse width: | 1-500 µs |

Burst stimulation parameters for detrusor and external urethral sphincter relaxation -human model

| | |
|---|---|
| Frequency: | 100-20000 Hz |
| Amplitude: | 0-50 mA |
| Pulse width: | 1-500 µs |

In humans, the high-frequency electrical stimulation for external urethral sphincter relaxation is preferably delivered according to one or more burst stimulation patterns including 0 to 20 pulses per burst, with pulse width between 1 and 500 µs and amplitude between 0 and 50 mA.

The connection between the control unit 12 and the stimulation unit 14 can be established by a wireless link.

As an alternative, the control unit 12 and the stimulation unit 14 can be operatively connected through a cable bound and/or unidirectional and/or indirect connection.

In the present embodiment, the control unit 12 is configured and arranged to control the stimulation unit 14 to provide electrical stimulation over a predefined period of time.

Advantageously, the predefined period of time can be specifically tuned based on specific conditions of a subject.

In particular, the predefined period of time ranges between 0 and 5 minutes.

Stimulation timing may be defined based on specific needs and/or preferences of a subject.

For instance, in case a subject wishes to extend a time period between a bladder voiding operation and the subsequent one, the amount of time of detrusor relaxation stimulation can be extended accordingly.

Further, a bladder voiding operation can be voluntarily triggered upon providing a specific user command.

For instance, in case a subject affected by bladder disfunction after SCI and/or other neurodegenerative and/or neurological disorders wishes to go out and avoid the need for bladder voiding while being out, he/she can trigger bladder voiding stimulation at before leaving home.

In the shown example, the system 10 is a closed-loop system.

In particular, the system 10 includes a sensor unit 16 (Fig. 1).

Not shown is that the sensor unit 16 may include one or more biocompatible implantable sensors.

The sensor unit 16 may be configured and arranged to detect one or more physiological values of said mammal, in particular a human, among:
- intra-bladder pressure;
- detrusor contraction, and/or
- external urethral sphincter pressure.

In the present embodiment, the sensor unit 16 is configured and arranged to transmit the one or more detected physiological values to the control unit 12.

In the present embodiment, the control unit 12 is configured and arranged to operate the stimulation unit 14 in a closed loop, based on the one or more detected physiological values from the sensor unit 16.

A method for stimulating neuronal circuitry responsible for micturition control in a mammal with bladder disfunction, especially for bladder relaxation and/or for bladder voiding, by using the neuromodulation/neurostimulation system 10 of the invention will be described in the following.

In particular, said mammal may be a human affected by SCI, other neurological disorders such as a stroke, multiple sclerosis, autonomic failure, autonomic neuropathy, other neurodegenerative disorders, or cancer of neurological tissue, which impair operation of descending sympathetic pathways that normally facilitate control of autonomic functions.

In the present example, the method includes the following steps of:
- positioning at least one stimulation unit on the body of a mammal, in particular a human, to deliver stimulation to the spinal cord of said mammal;
- delivering low-frequency electrical stimulation to the last lumbar spinal segment and/or the first two sacral segments of the spinal cord of said mammal, especially at level L5-S1 of the spinal cord, to stimulate autonomic neurons responsible for detrusor contraction, and
- simultaneously deliver high-frequency electrical stimulation at sacral level, especially to the first three sacral segments of the spinal cord of the spinal cord of said mammal, to modulate sacral motoneurons responsible for external urethral sphincter relaxation.

In particular, the method includes delivering the low-frequency electrical stimulation for detrusor contraction at a frequency between 10 and 700 Hz.

Preferably, the method includes delivering the low-frequency electrical stimulation for detrusor contraction at a frequency between 10 and 500 Hz.

More preferably, the method includes delivering the low-frequency electrical stimulation for detrusor contraction at a frequency between 10 and 200 Hz.

Advantageously, the method includes delivering the low-frequency electrical stimulation for detrusor contraction with an amplitude between 0 and 50 mA.

Advantageously, the method includes delivering the low-frequency electrical stimulation for detrusor contraction with a pulse width between 1 and 50 µs.

In humans, the method preferably includes delivering the low-frequency electrical stimulation for detrusor contraction at a frequency between 10 and 500 Hz with amplitude between 0 and 50 mA and pulse width between 1 and 50 µs.

In humans, the method more preferably includes delivering the low-frequency electrical stimulation for detrusor contraction at a frequency between 10 and 200 Hz with amplitude between 0 and 50 mA and pulse width between 1 and 50 µs.

As an alternative, the method includes delivering the low-frequency electrical stimulation for detrusor contraction according to one or more burst stimulation patterns.

Preferably, the one or more burst stimulation patterns include 0 to 20 pulses per burst.

In the described method, the one or more burst stimulation patterns have a carrier frequency between 10 and 700 Hz.

Preferably, the one or more burst stimulation patterns have a carrier frequency between 0 to 500 Hz.

More preferably, the one or more burst stimulation patterns have a carrier frequency between 0 to 200 Hz.

Advantageously, the one or more burst stimulation patterns have burst frequency between 100 and 5000 Hz.

Advantageously, the one or more burst stimulation patterns have an amplitude between 0 and 50 mA.

In a human subject, the method preferably includes delivering the low-frequency electrical stimulation for detrusor contraction according to one or more burst stimulation patterns including 0 to 20 pulses per burst, with carrier frequency between 10 and 500 Hz, burst frequency between 100 and 5000 Hz, and amplitude between 0 and 50 mA.

In a human subject, the method more preferably includes delivering the low-frequency electrical stimulation for detrusor contraction according to one or more burst stimulation patterns including 0 to 20 pulses per burst, with carrier frequency between 10 and 200 Hz, burst frequency between 100 and 5000 Hz, and amplitude between 0 and 50 mA.

The method includes delivering the high-frequency electrical stimulation for external urethral sphincter relaxation at a frequency between 100 and 20000 Hz.

Preferably, the method includes delivering the high-frequency electrical stimulation for external urethral sphincter relaxation at a frequency of 10000 Hz.

Advantageously, the method includes delivering the high-frequency electrical stimulation for external urethral sphincter relaxation according to one or more burst stimulation patterns.

Preferably, the one or more burst stimulation patterns include 0 to 20 pulses per burst.

Advantageously, the one or more burst stimulation patterns have a pulse width between 1 and 500 µs.

Advantageously, the one or more burst stimulation patterns have an amplitude between 0 and 50 mA.

In humans, the method preferably includes delivering the high-frequency electrical stimulation for external urethral sphincter relaxation according to one or more burst stimulation patterns including 0 to 20 pulses per burst, with pulse width between 1 and 500 µs, and amplitude between 0 and 50 mA.

The method may further include:
- detecting one or more physiological values of said mammal, in particular a human, through a sensor unit 16;
- transmitting the one or more detected values to a control unit 12, and
- operating the stimulation unit 14 based on the one or more detected values from the sensor unit 16.

The sensor unit 16 may include one or more biocompatible implantable sensors.

The physiological values of said mammal, in particular a human, may include one or more among:
- intra-bladder pressure;
- detrusor contraction, and/or
- external urethral sphincter pressure.

The electrical stimulation is provided over a predefined period of time.

The predefined period of time may be specifically tuned based on specific conditions of a subject.

In particular, the predefined period of time ranges between 0 and 5 minutes.

### Exemplary system setups

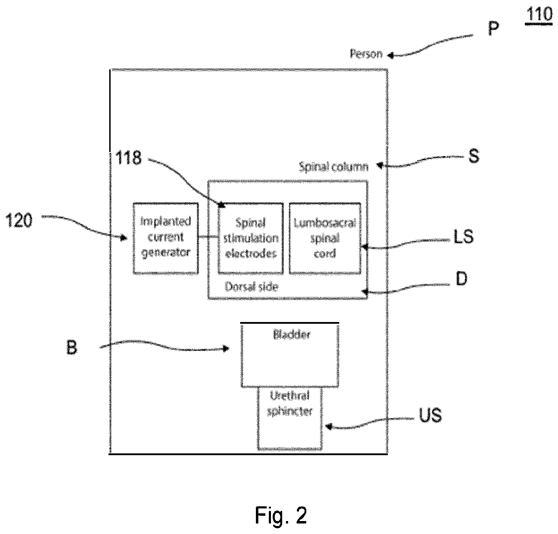

**Fig. 2** shows an exemplary setup for a neuromodulation/neurostimulation system 110 for bladder relaxation.

The system 110 includes an implanted current generator 120 connected to one or more spinal stimulation electrodes 118 epidurally implanted over the spinal column S of a patient P, especially over the dorsal side D of the lumbosacral spinal cord LS to relax the bladder B.

In this example, the implanted current generator 120 is connected to the one or more spinal stimulation electrodes 118 through cables and is configured to deliver electrical current through their contacts. The implanted current generator 120 can generate a temporal sequence of electrical currents on each of said contacts. Therefore, the implanted current generator 120 delivers the current through its contacts, over the cable and then through the one or more spinal stimulation electrodes 118.

The implanted current generator 120 can work independently until its batteries have been discharged. The implanted current generator 120 may be rechargeable. Further, the implanted current generator 120 may be controlled by another device (not shown) that can modify the sequence of current delivery, including stopping the delivery of the current.

Optionally, the patient P may be provided with a feedback signal relating to the delivered spinal stimulation by different modalities, including but not limited to sound, touch, vibration and visual stimuli.

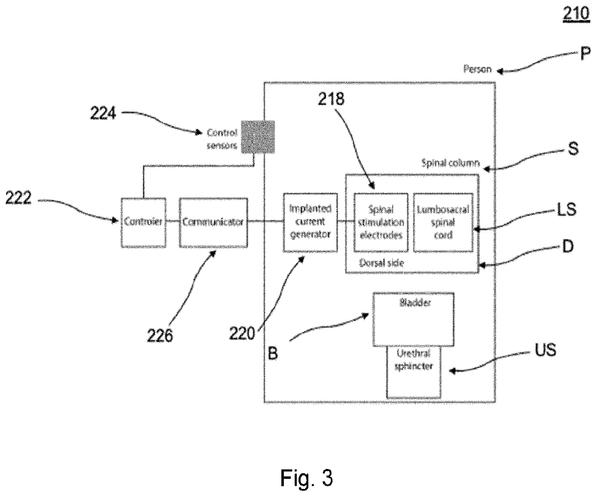

**Fig. 3** is a diagram showing an exemplary setup for a neuromodulation/neurostimulation system 210 that is similar to the one of **Fig. 2****,** but where the system 210 is further equipped with one or more control sensors 224.

In particular, the one or more control sensors 224 are configured to send detected signals to a controller 222 through wired and/or wireless links. The controller 222 uses the acquired signals to derive the stimulation commands, and then send these commands to a communicator 226. The communicator 226 relays the commands to an implanted current generator 220.

The implanted current generator 220 is connected to one or more spinal stimulation electrodes 218 epidurally implanted over the spinal column S of a patient P, especially over the dorsal side D of the lumbosacral spinal cord LS, and is configured to deliver spinal cord stimulation in the same way as the system 110 described above with reference to **Fig. 2****.**

The one or more control sensors 224 may be configured to collect one or more user's commands provided through a user input device (not shown) such as a button or a device configured for receiving a vocal command.

The one or more control sensors 224, the controller 222 and/or the communicator 226 may be advantageously integrated in a single device.

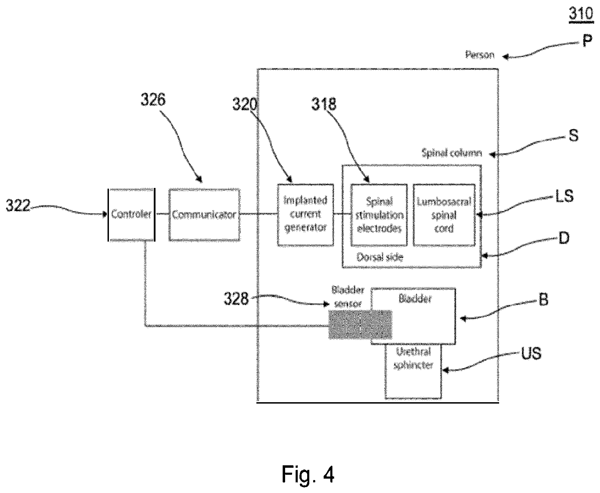

**Fig. 4** is a diagram showing an exemplary setup for a neuromodulation/neurostimulation system 310 that is similar to the one of **Fig. 2****,** but where the system 310 is further equipped with one or more bladder sensors 328 implanted in the bladder B of the patient P.

The one or more bladder sensors 328 send detected signals to a controller 322 through wired and/or wireless links. The controller 322 uses the acquired signals to infer the state of the bladder B of the patient P, derives stimulation commands, and sends these commands to a communicator 326. Then, the communicator 326 relays the commands to an implanted current generator 320.

The implanted current generator 320 is connected to one or more spinal stimulation electrodes 318 epidurally implanted over the spinal column S of a patient P, especially over the dorsal side D of the lumbosacral spinal cord LS, and is configured to deliver spinal cord stimulation in the same way as the system 110 described above with reference to **Fig. 2****.**

In particular, the one or more bladder sensors 328 are configured to measure a state of the bladder B, including but not limited to bladder pressure and/or detrusor muscle contraction.

The one or more bladder sensors 328, the controller 322 and/or the communicator 326 may be advantageously integrated in a single device.

The features of systems 110, 210 and 310 described above with reference to **Figs. 2** to **4** may as well be incorporated in a single system (not shown).

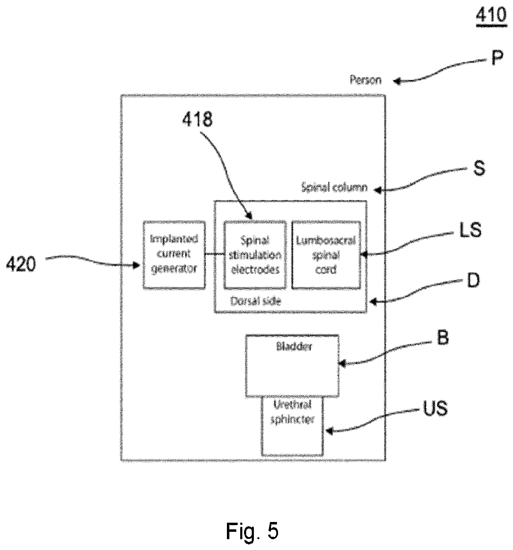

**Fig. 5** is a diagram showing an exemplary setup for a neuromodulation/neurostimulation system 410 for bladder voiding.

The system 410 includes an implanted current generator 420 connected to one or more spinal stimulation electrodes 418 epidurally implanted over the spinal column S of a patient P, especially over the dorsal side D of the lumbosacral spinal cord LS to void the bladder B.

In this example, the implanted current generator 420 is connected to the one or more spinal stimulation electrodes 418 through cables and is configured to deliver electrical current through their contacts. The implanted current generator 420 can generate a temporal sequence of electrical currents on each of said contacts. Therefore, the implanted current generator 420 delivers the current through its contacts, over the cable and then through the one or more spinal stimulation electrodes 418.

The implanted current generator 420 can work independently until its batteries have been discharged. The implanted current generator 420 may be rechargeable. Further, the implanted current generator 120 may be controlled by another device (not shown) that can modify the sequence of current delivery, including stopping the delivery of the current.

Optionally, the patient P may be provided with a feedback signal relating to the delivered spinal stimulation by different modalities, including but not limited to sound, touch, vibration and visual stimuli.

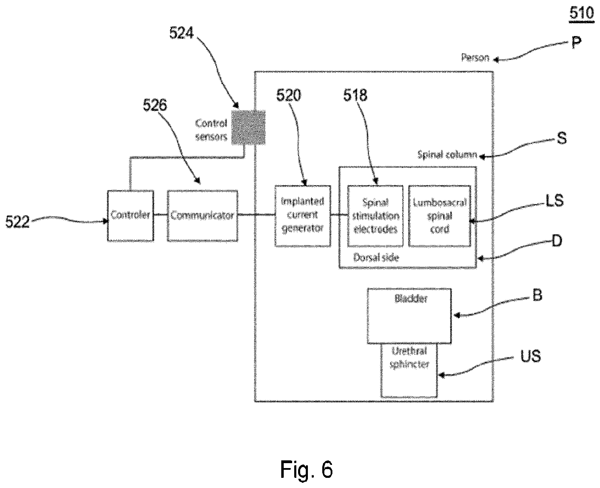

**Fig. 6** is a diagram showing an exemplary setup for a neuromodulation/neurostimulation system 510 that is similar to the one of **Fig. 5****,** but where the system 510 is further equipped with one or more control sensors 524.

The one or more control sensors 524 send detected signals to a controller 522 through wired and/or wireless links. The controller 522 uses the acquired signals to derive the stimulation commands, and send these commands to a communicator 526. Then, the communicator 526 relays the commands to an implanted current generator 520.

The implanted current generator 520 is connected to one or more spinal stimulation electrodes 518 epidurally implanted over the spinal column S of a patient P, especially over the dorsal side D of the lumbosacral spinal cord LS, and is configured to deliver spinal cord stimulation in the same way as the system 410 described above with reference to **Fig. 5****.**

The one or more control sensors 524 may be configured to collect one or more user's commands provided to a user input device (not shown) such as a button or a device configured for receiving a vocal command.

The one or more control sensors 528, the controller 522 and/or the communicator 526 may be advantageously integrated in a single device.

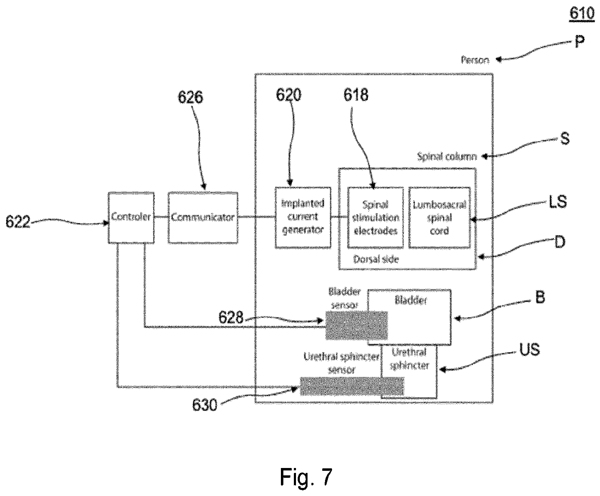

**Fig. 7** is a diagram showing an exemplary setup for a neuromodulation/neurostimulation system 610 that is similar to the one of **Fig. 5****,** but where the system 610 is further equipped with one or more bladder sensors 628 implanted in the bladder B of the patient P and/or one or more urethral sphincter sensors 630 implanted in the urethral sphincter US of the patient P.

The one or more bladder sensors 628 and/or the one or more urethral sphincter sensors 630 send the signals to a controller 622 through wired and/or wireless links. The controller 622 uses the acquired signals to derive the stimulation commands, and send these commands to a communicator 626. The communicator 626 relays the commands to an implanted current generator 620.

The implanted current generator 620 is connected to one or more spinal stimulation electrodes 618 epidurally implanted over the spinal column S of a patient P, especially over the dorsal side D of the lumbosacral spinal cord LS, and is configured to deliver spinal cord stimulation in the same way as the system 410 described above with reference to **Fig. 5****.**

In particular, the one or more bladder sensors 628 are configured to measure a state of the bladder B, including but not limited to bladder pressure and/or detrusor muscle contraction, while the one or more urethral sphincter sensors 630 are configured to measure urethral sphincter contraction.

The one or more bladder sensors 628, the one or more urethral sphincter sensors 630, the controller 622 and/or the communicator 626 may be advantageously integrated in a single device.

The features of systems 410, 510 and 610 described above with reference to **Figs. 5** to **7** may as well be incorporated in a single system (not shown).

### Experimental setup for bladder relaxation

In the following, an experimental setup for a neuromodulation/neurostimulation device for bladder relaxation in a patient with chronic spinal cord injury will be described.

The system is configured for delivery of epidural electrical stimulation of the lumbosacral spinal cord controlled by an invasive bladder pressure sensor, e.g. as described above with reference to **Fig. 4****.**

In particular, the system includes:
- an implanted current generator 320 - e.g., Activa RC IPG (Medtronic).
- electrodes - e.g., one 16-electrode Specify 5-6-5 lead (Medtronic) implanted over the dorsal side of the lumbosacral spinal cord.
- a bladder sensor 328 - one bladder pressure sensor inserted into the bladder.
- a controller 322 - a wireless receiver connected to a data acquisition system that is wired to a computer equipped with a Bluetooth wireless transmitter.
- a communicator 326 - a Patient Programmer that receives the stimulation commands via Bluetooth and relays them to the implanted current generator via wireless telemetry.

The implanted current stimulator is programmed with a bladder relaxation EES protocol. This protocol is defined by the set of spinal electrodes that delivers the stimulation current, current amplitude, pulse waveform, and stimulation frequency.

These protocols are designed as follows.

The patient P is seated comfortably, while the treating physician uses the system software to identify the parameters of the protocol. The system 310 uses a reinforcement learning algorithm to iteratively trigger EES pulses using different sets of EES parameters (stimulation amplitude, frequency, electrode configuration) and analyze the bladder pressure responses. By seeking the stimulation that maximally reduces the bladder spasms, this procedure converges to a set of EES parameters that define the bladder relaxation EES protocol.

The bladder relaxation EES program is loaded into the firmware of the implanted current generator. The EES is then activated. The bladder pressure sensor measures the pressure in the bladder and sends its signals to the controller. The controller compares the current value of the bladder pressure against the bladder pressure target set by the user or by the treating physician. When the measured pressure goes above the target, the controller sends a command to the communicator to activate the bladder relaxation EES protocol. The communicator then relays this command to the implanted current generator. When the measured pressure goes below the target, the controller sends a command to the communicator to deactivate the bladder relaxation EES protocol. The bladder relaxation EES protocol may be modified during subsequent re-calibration.

### Experimental setup for bladder voiding

In the following, an experimental setup for a neuromodulation/neurostimulation device for bladder voiding in a patient with chronic spinal cord injury will be described.

The system is configured for delivery of epidural electrical stimulation of the lumbosacral spinal cord controlled by an invasive bladder pressure sensor and an invasive urethral sphincter sensor, e.g. as described above with reference to **Fig. 7****.**

In particular, the system includes:
an implanted current generator 620 - e.g. Activa RC IPG (Medtronic).
spinal electrodes -e.g. one 16-electrode Specify 5-6-5 lead (Medtronic) implanted over the dorsal side of the lumbosacral spinal cord.
a bladder sensor 628 - one bladder pressure sensor inserted into the bladder.
a urethral sphincter sensor 630 - one EMG sensor placed at the ureteral sphincter.
a controller 622 - a wireless receiver connected to a data acquisition system that is wired to a computer equipped with a Bluetooth wireless transmitter.
a communicator 626 - a Patient Programmer that receives the stimulation commands via Bluetooth and relays them to the implanted current generator via wireless telemetry. The implanted current stimulator 620 is programmed with two programs: (i) bladder relaxation tonic program and (ii) bladder voiding sequence. The two programs are composed of four EES protocols: (i) detrusor muscle relaxation, (ii) detrusor muscle contraction, (iii) urethral sphincter relaxation, (iv) urethral sphincter contraction. Each of these protocols is defined by the set of spinal electrodes that delivers the stimulation current, current amplitude, pulse waveform, and stimulation frequency.

These protocols are designed as follows.

The user (patient/person P) is seated comfortably, while the treating physician uses the system software to identify the parameters of the protocol. The system 610 uses a reinforcement learning algorithm to iteratively triggers EES pulses using different sets of EES parameters (stimulation amplitude, frequency, electrode configuration) and analyze the bladder pressure response for protocols (i) and (ii), and urethral sphincter EMG for protocols (iii) and (iv). This procedure converges to a set of EES parameters that define the EES protocol by seeking the stimulation that, for (i) maximally reduces the bladder spasms, for (ii) maximally increases the bladder pressure, for (iii) maximally increases urethral sphincter EMG and, for (iv) maximally reduces urethral sphincter EMG.

The bladder relaxation tonic program continuously delivers the bladder relaxation and urethral sphincter contraction EES protocols. The bladder voiding sequence comprises first the period of bladder contraction and urethral sphincter contraction EES protocol co-activation. Once the bladder pressure reaches the "bladder voiding" threshold set by the user or the treating physician, the system transitions into the period of coactivation of the bladder contraction and urethral sphincter relaxation EES protocols. Once the bladder pressure reaches another "bladder closure" threshold, the system transitions into the bladder relaxation tonic program.

The bladder relaxation tonic program and bladder voiding sequence are loaded into the firmware of the implanted current generator. The bladder relaxation tonic program is then activated. The bladder pressure sensor measures the pressure in the bladder and sends its signals to the controller - a control computer. The controller compares the current value of the bladder pressure against the bladder voiding threshold set by the user or by the treating physician. When the measured pressure goes above the threshold, the controller sends a command to the communicator to activate the bladder voiding sequence. communicator then relays this command to the implanted current generator. When the measured pressure goes below the bladder closure threshold after the voiding, the controller sends a command to the communicator to activate the bladder relaxation tonic program. All of the EES protocols may be modified during subsequent re-calibration.

### Experimental model in rats

Experiments on rodents, specifically rats, have been carried out by the inventors to define optimal stimulation sites on the spinal cord for modulating detrusor and urethral sphincter activity.

Specifically, a functional mapping procedure has been carried out on a rat model of thoracic SCI to identify the optimal location on the spinal cord to elicit a bladder voiding response.

In the functional mapping procedure, each segment of the spinal cord from T11 to S4 has been stimulated.

Intra-bladder pressure response and urethral sphincter electromyogram have been recorded.

A monopolar low-frequency electrical stimulation at 50 Hz has been applied to spinal cord segments T11 to S4 of rats having thoracic SCI for detrusor muscle contraction.

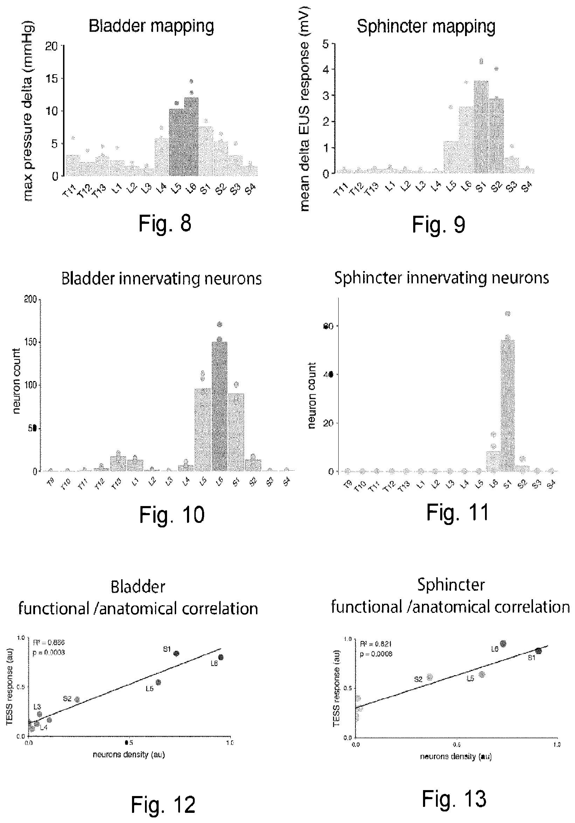

**Fig. 8** is a diagram showing functional mapping results in rats having thoracic SCI after stimulating each segment of the spinal cord from T11 to S4 with monopolar low-frequency stimulation for detrusor contraction.

Based on this experimental evidence, the inventors have found out that spinal cord segments L5-L6 represent the optimal stimulation site for contraction of the detrusor muscle, induced with low-frequency stimulation at 50Hz (**Fig. 8**).

High-frequency electrical stimulation at 5000Hz has been applied to spinal cord segments T11 to S4 of rats having thoracic SCI for external urethral sphincter relaxation.

**Fig. 9** is a diagram showing functional mapping results in rats having thoracic SCI after stimulating each segment of the spinal cord from T11 to S4 with high-frequency stimulation at 5000Hz for external urethral sphincter relaxation.

Based on this experimental evidence, the inventors have found out that spinal cord segments S1-S2 represent the optimal stimulation site for external urethral sphincter relaxation induced with high-frequency stimulation at 5000Hz (**Fig. 9**).

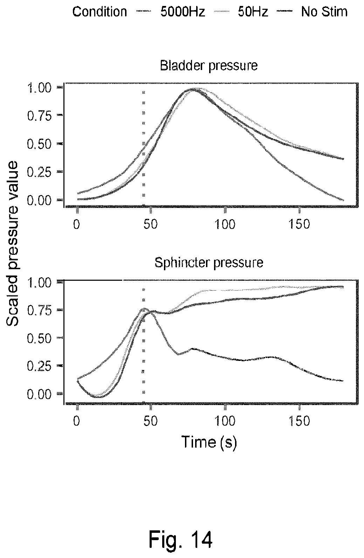

**Fig. 14** illustrates a diagram showing external urethral sphincter silencing during bladder filling with induced sphincter silencing using electrical stimulation at 5000Hz at spinal cord level S1. The dotted lines denote the moment in which stimulation has been turned on.

Next, density of autonomic neurons and motoneurons in the spinal cord projecting respectively to the bladder wall and the urethral sphincter has been determined.

**Fig. 10** is a diagram showing the results of retrograde tracing from the bladder wall by counting labeled spinal neurons for each segment of the spinal cord from T9 to S4.

**Fig. 11** is a diagram showing results of retrograde tracing from the urethral sphincter muscle by counting labeled spinal neurons for each segment of the spinal cord from T9 to S4.

Based on this experimental evidence, the inventors have found out that the density of these populations of neurons peaked at spinal cord level L6 for detrusor contraction (**Fig. 10**) and on spinal cord level S1 for external urethral sphincter relaxation (**Fig**. **11**).

The inventors have also found out that there is a significant linear correlation between these findings and the results of functional mapping.

The linear correlation between bladder functional and anatomic mappings in the analyzed rat model is shown in **Fig. 12****.**

The linear correlation between external urethral sphincter functional and anatomic mappings in the analyzed rat model is shown in **Fig. 12****.**

Summarizing, the inventors have found out that spinal cord segments L5-L6 are the optimal sites to stimulate for detrusor contraction (**Fig. 8**), with peaks occurring during stimulation of spinal cord segment L6 (**Fig. 10**), while spinal cord segments S1-S2 are the optimal sites to stimulate for external urethral sphincter relaxation (**Fig. 9**), with peaks occurring during stimulation of spinal cord segment S1 (**Fig**. **11**)**.**

With the knowledge that these two spinal localizations preferentially activate neuronal structures that are responsible for detrusor and external urethral sphincter activity, high resolution CT and MRI scans have been performed to accurately define the relationship between spinal segments and vertebral levels.

Additionally, ex vivo dissection confirmed the exact length of L5-L6 and S1-S2 segments.

Based on the above mentioned experimental results, a biocompatible spinal implant has been specifically designed and dimensioned to stimulate spinal cord segments L5-L6 and S1-S2 through electrode arrays implanted immediately under the T13-L1-L2 vertebra.

This array design may be easily scaled and adapted to any animal or human model by using MRI technology and computational modelling.

Further, a stimulation paradigm has been designed that mimics the natural state of the intact nervous system innervating low urinary tractus.

Specifically, low frequency oscillation overlays originating in supraspinal structures responsible for intra-bladder pressure and sphincter relaxation control (i.e., the pontine micturition center) have been optimized, and a 0.1 Hz low-frequency oscillation in the amplitude or frequency control of the stimulation has been input.

The stimulation paradigm reproduces the natural supraspinal brainstem center drive and, when coupled with a range of standard parameters, achieves the best control over urodynamic functions after spinal cord injury or other neurodegenerative or neurological disorder.

This biomimetic stimulation may either be triggered through a closed-loop system based on an input from a biosensor (e.g., detecting intra-bladder pressure), or may be triggered volitionally by providing a specific user command.

Additionally or alternatively, the above disclosure may be combined with the following concept of spinal epidural electrical stimulation therapy to alleviate bladder-related deficits of spinal cord injury.

### Patient data

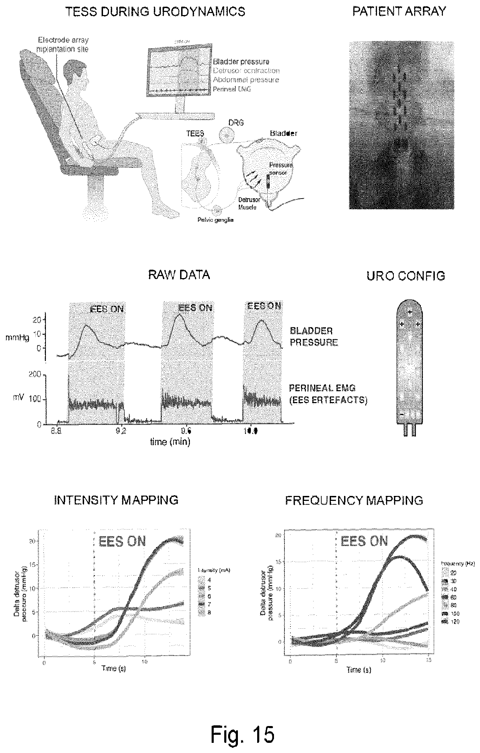

Parameters of epidural electrical stimulation (EES) for bladder contraction have been successfully identified in one person with a thoracic spinal cord injury. The subject has been implanted by a device that includes an EES lead inserted in the dorsal aspect of the epidural space at the lumbo-sacral level of the spinal cord (**Fig. 15**). During urodynamic assessments, it was shown that EES targeting the dorsal roots of the final lumbar spinal cord segments induced consistent and persistent contractions of the detrusor muscle, which contracts the bladder.

The responses of the detrusor muscle were mapped to the EES of varying frequency, amplitude and electrode configurations in two separate urodynamic assessment exams 8 months apart. The same optimal frequency, amplitude and electrode configuration was found in both of these sessions.

Required input: The required input to optimize the EES parameters are the signals of an intra-bladder pressure sensor and an intra-urethral sphincter pressure sensor. A medical specialist inserts both of these sensors during standard urodynamic exams. After insertion, the specialist will use these sensors to identify "resting" pressure of both organs.

Spatial mapping phase: A software is used to take the clinician or researcher through a series of steps to isolate the electrode configuration that most readily increases intra-bladder and intra-urethral sphincter pressure, with minimal effects on other systems (e.g., minimal muscle activity). This configuration is dependent on the exact location of the spinal EES lead. For a lead of 16 electrodes and three potential states of the electrode in a given configuration (anode, cathode, null), there are more than 40 million possible configurations. This expansive parameter space is narrowed by using computational simulations on a personalized bioelectrical model of the spine. This model is generated from 3T MRI and CT scans of the subject. The simulations are used to determine the optimal configuration of the electrodes to stimulate the posterior roots responsible for regulation of bladder voiding. Reinforcement learning procedure is used to identify the electrode configuration of the electrodes for the bladder contraction. This procedure selects the EES configuration and then elicits a brief EES episode. The reinforcement learning algorithm monitors the pressure and calculates a 'reward' proportional to an increase in pressure. The algorithm then looks for EES configuration that maximizes the reward, i.e. elicit largest pressure increase. The specialist can intervene to reduce the reward associated with a set of parameters if the patient finds EES with those parameters uncomfortable, or if they notice other side effects (e.g., spasms). The algorithm moves through the space of EES configurations to identify the optimal one for bladder contraction. The same procedure was used to identify the optimal electrode configuration for the urethral sphincter relaxation.

Parameter mapping phase: Next, the software uses reinforcement learning procedure to identify effective EES pulse width (range 270 - 450 us) and frequency (50 - 120 Hz).

### Restoration of the ability to void the bladder

Following the identification of EES protocols for the three responses, bladder relaxation, bladder contraction and urethral sphincter relaxation, those protocols were used to achieve three efficient episodes of bladder voiding (**Fig. 15**).

### Prevention of pathologic bladder contractions

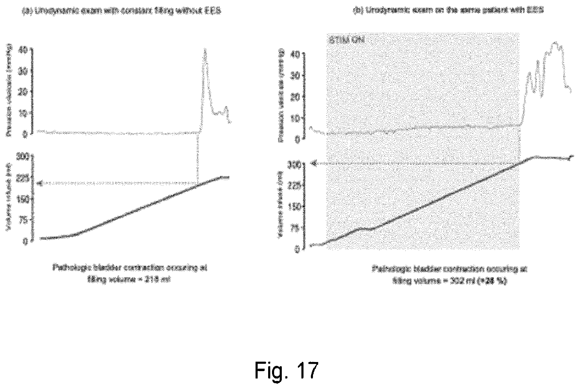

In a chronic state of spinal cord injury, the reorganization of the neuronal pathways below the injury provokes involuntary bladder contractions leading to urinary incontinence. Filling a bladder during a urodynamic exam causes bladder wall distention, which can evoke these pathologic bladder contractions. Using high frequency EES delivered at the L5/S1 spinal level, bladder contraction was prevented at a higher filling volume. Turning the EES off immediately triggered the bladder contraction (**Fig. 17**). The prevention of contractions during EES and the immediate triggering of the contractions when the EES is turned off consolidates the direct effect of this technology on the urodynamic function.

### Bladder stimulation parameters

A stimulation paradigm was designed that mimics the natural bladder voiding response that can be recorded during a urodynamic exam in people with spinal cord injury. To do this a series of stimulation features was combined that collectively accomplish four tasks:
- Relax the detrusor muscle
- Contract the detrusor muscle
- Relax the external urethral sphincter
- Contract the external urethral sphincter

The timing of these stimulation features can be custom tuned. For example, if one wishes to extend the period of time between voids, one can increase the amount of time of detrusor relaxation stimulation. If one wishes to induce a void on command, for example in a person with spinal cord injury that wants to go out for dinner but not need to void while at the restaurant, one can just trigger the voiding stimulation program.

The voiding stimulation program consists of a rapid increase in detrusor muscle stimulation achieved using EES with the following parameters: Frequency: 10-500Hz, Amplitude: 0-50mA, Pulse width: 1-500us. Similar response can be achieved using EES burst-stimulation patterns with the following parameters: number of pulses per burst = 0-20; carrier frequency = 10-500Hz; burst frequency = 100-5000Hz; Amp: 0-50mA. This stimulation is delivered over a set period of time, x, which can be tuned to each person (ranging from 0-5 min). The stimulation can be either triggered as ON / OFF, or delivered according to the exponential rise in detrusor pressure found in the natural bladder voiding response. Thus, the stimulation amplitude or frequency can be increased as a function of an exponential curve, or more broadly any polynomial curve.

Relaxation of the detrusor and external urethral sphincter can be accomplished using burst or high frequency EES patterns with the following parameters: Frequency: 100-20000Hz, Amplitude: 0-50mA, Pulse width: 1-500us.

Together, these features can then be controlled in closed-loop, according to bladder pressure, detrusor activity, external urethral sphincter pressure or activity, or some combination of these.

### Pre-clinical data

### Bladder relaxation

To identify the optimal EES target on the spinal cord to modulate the bladder activity, the elevation of the intra-bladder pressure was measured, resulting from the detrusor muscle contraction, in response to EES of each spinal cord segment from T11 to S4. Applying this procedure on a mouse model of thoracic spinal cord injury with a monopolar 50Hz stimulation, it was found that L5-L6 are the optimal segments for an efficient contraction of the detrusor muscle **(****Fig. 18a****).** Next, the density of autonomic neurons and motoneurons in the spinal cord projecting to the bladder wall was determined. It was found that the density of these populations of neurons peaked on L6 **(****Fig. 18b****).** A strong linear correlation was observed between the density of autonomic neurons and the elevation of the intra-bladder pressure achieved by EES **(****Fig. 18c****).**

Then the intra-bladder pressure elevation was mapped in response to L6 EES of frequencies spanning the 50Hz to 5000Hz range. High frequency EES reduced the amplitude of aberrant bladder contractions **(****Fig. 18d****-e).** It was found that both open loop and closed loop EES significantly reduces the severity of bladder overactivity in a chronic phase of SCI.

### Restoring urodynamic function

### Identification of the optimal spinal level

The neuronal pathways involved in bladder voiding and a functional mapping throughout the spinal cord were traced. Pseudorabies virus encoding for a fluorescent protein was injected directly into the bladder wall and the urethral sphincter to label retrogradely the spinal neurons that project to these targets (**Fig. 19a**). It was found that the density of pre-ganglionic neurons projecting to the detrusor muscle of the bladder peaked at L6 segment, while somatic motor neurons projecting to the urethral sphincter peaked at S1 segment (**Fig. 19b**). Furthermore, the tri-dimensional organization of these neurons was characterized through a translucid central nervous system using tissue clearing technology (**Fig. 19c**). Then, a mapping of the spinal cord was performed by applying EES segment per segment while recording pressure into the bladder and an electromyogram of the urethral sphincter (**Fig. 20a**). The contraction of these muscles was maximum at similar levels, leading to a strong anatomical-functional correlation for both muscles (**Fig. 20b****-c**).

### Design of the neuroprosthesis

Based on the results of the mapping, epidural electrode arrays were developed and fabricated. These arrays deliver current through stretchable gold tracks covered by a highly biocompatible material (**Fig. 21a**). The geometry of electrode distribution on the design enables the recruitment of L5, L6, S1 and S2 segments with a high specificity using a current steering strategy. The arrays were first implanted in healthy wildtype rats. In these animals, it was demonstrated that a biomimetic EES sequence induces successful bladder contractions and sphincter relaxation through the appropriate roots (**Fig**. **21b**)**.** These arrays were then implanted in a T9 transection rodent spinal cord injury model. It was shown that a neuroprosthesis based on the biomimetic EES sequence can induce regular bladder voidings and, therefore, restore urodynamic function after SCI (**Fig. 21c**).

### Bladder stimulation parameters

A stimulation paradigm was designed that mimics the natural bladder voiding response that can be recorded during a urodynamics session in intact animals (as illustrated in **Fig. 14**). To do this, a series of stimulation features was combined that collectively accomplish four tasks:
- Relax the detrusor muscle
- Contract the detrusor muscle
- Relax the external urethral sphincter
- Contract the external urethral sphincter

The timing of these stimulation features can be custom tuned. For example, if one wishes to extend the period of time between voids, one can increase the amount of time of detrusor relaxation stimulation. If one wishes to induce a void on command, for example in a person with spinal cord injury that wants to go out for dinner but not need to void while at the restaurant, one can just trigger the voiding stimulation program.

The voiding stimulation program consists of a rapid increase in detrusor muscle stimulation achieved using EES with the following parameters:
- Mice and rats: Freq: 10-700Hz, Amp: 0-2000µA, Pulse width: 1-500us
- Non-human primates: Freq: 10-500Hz, Amp: 0-50mA, Pulse width: 1-500us

Similar response can be achieved using EES burst-stimulation patterns with the following parameters:
- Mice and rats: number of pulses per burst = 0-20, carrier frequency = 10-700Hz, burst frequency = 100-5000Hz, Amp: 0-2000µA
- Non-human primates: number of pulses per burst = 0-20; carrier frequency = 10-500Hz; burst frequency = 100-5000Hz; Amp: 0-50mA

This stimulation is delivered over a set period of time, x, which can be tuned to each person (ranging from 0-5 min). The stimulation can be either triggered as ON / OFF, or delivered according to the exponential rise in detrusor pressure found in the natural bladder voiding response. Thus, the stimulation amplitude or frequency can be increased as a function of an exponential curve, or more broadly any polynomial curve.

Relaxation of the detrusor and external urethral sphincter can be accomplished using burst or high frequency EES patterns with the following parameters:
- Mice and rats: Freq: 100-20000Hz, Amp: 0-2000µA, Pulse width: 1-500us
- Non-human primates: Freq: 100-20000Hz, Amp: 0-50mA, Pulse width: 1-500us

Together, these features can then be controlled in closed-loop, according to bladder pressure, detrusor activity, external urethral sphincter pressure or activity, or some combination of these.

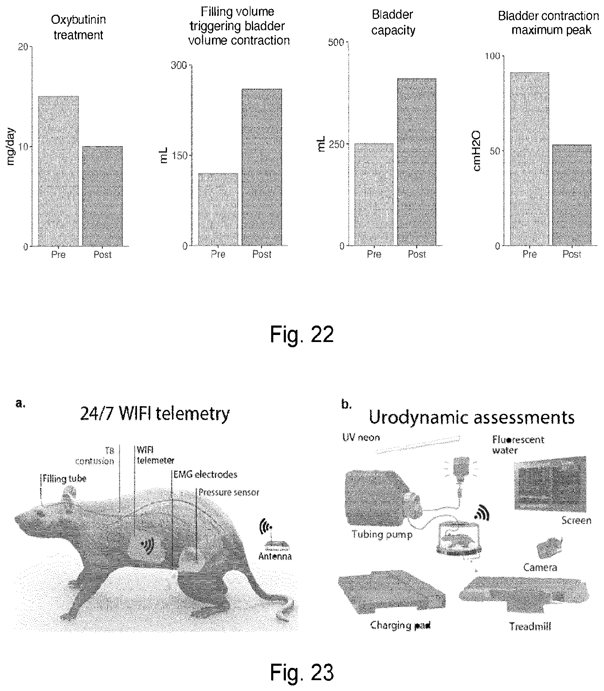

**Fig. 22** illustrates a diagram showing the long-term (rehabilitative) effects of stimulation. Accordingly, the bar graphs indicate an overall decrease in oxybutynin treatment accompanied by an increase in the volume required to trigger a bladder contraction during urodynamics, bladder capacity, and a decrease in the peak bladder contraction even when stimulation was turned off after several months of daily stimulation use.

### Continuous monitoring of the urinary function

To evaluate the potential therapeutic effect of chronic activation of the neurological circuitry involved in urinary function we elaborated a rehabilitation protocol on rodents. After a severe spinal cord injury, each rat was implanted with a neuroprosthesis targeting the urodynamic hotspot to restore the micturition reflex. Urinary function was recorded on each animal using a wireless telemetry device connected to a pressure sensor inserted into the bladder and EMG electrodes implanted in the urethral sphincter (**Fig. 23a**). The main urodynamic parameters, such as the micturition reflex frequency were measured in real time 24/7. Furthermore, we performed urodynamic assessments every week to calculate the micturition efficiency via the volume of each void (**Fig. 23b**).

### Establishment of a rehabilitation protocol

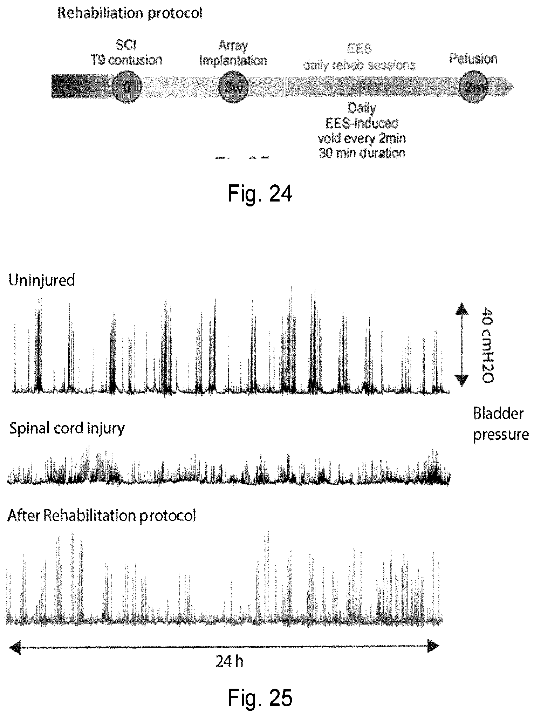

After undergoing a severe spinal cord injury, each rat was following a 3-week duration protocol where stimulation sessions were performed every day. During each session of 30 minutes duration, the micturition reflex was triggered by delivering EES using the neuroprosthesis every 2 minutes. The optimal parameters of the stimulation enabling to empty entirely the bladder were identified during the first rehabilitation session (**Fig. 24**).

### Improvement of the urinary function after the rehabilitation protocol

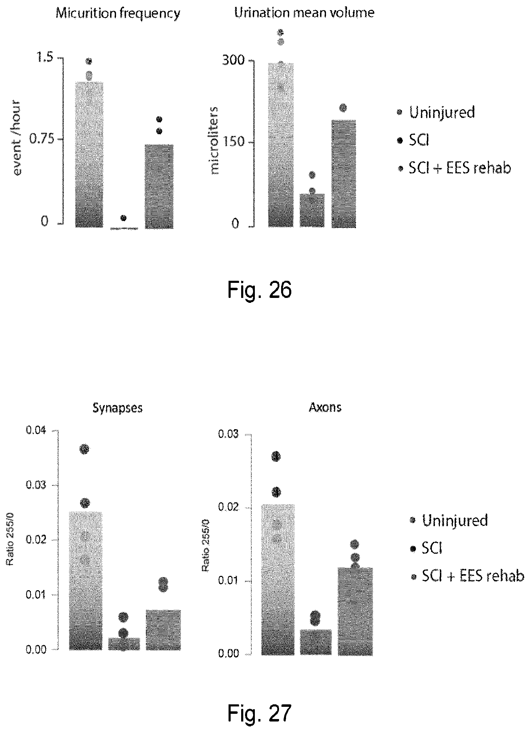

We quantified the main urodynamic parameters of a group of 4 rats which completed the rehabilitation protocol (SCI + rehab) and compared them to healthy rats (UI) and rats with spinal cord injury without rehabilitation (SCI). We observed a major improvement of the urinary function with an increase of the micturition reflexes and an elevation of the mean void volume (**Figs. 25****,** **26**).

### Reinforcement of the neurological projections from the Pontine Micturition Center

To confirm histologically the effect of the rehabilitation protocol we combined it to a study of the pathways involved in the micturition reflex. We used a combination of viral injections to trace and quantify the projections from the Pontine Micturition Center to the sacral spinal cord for each group. We observed an increase of these projections in terms of axons and synapses supporting their reinforcement in the rehabilitation group (SCI + rehab) compared to the group without rehabilitation (SCI) (**Fig. 27**).

These findings support the therapeutic effect of a chronic activation of the circuitry on the urinary function.

Moreover, the following aspects shall form a part of the disclosure of the present invention, either alone and/or in combination with at least one of the features as described above:
1. A neuromodulation/neurostimulation system (10) for stimulating at least one neuronal circuitry responsible for micturition control in a mammal with bladder disfunction, especially for bladder relaxation and/or for bladder voiding, said system (10) comprising:
   - at least one control unit (12), and
   - at least one stimulation unit (14), configured and arranged to provide electrical stimulation to the spinal cord of said mammal.
2. The system according to aspect 1, characterized in that the at least one control unit (12) is configured and arranged to control the at least one stimulation unit (14) to provide low-frequency electrical stimulation to the last lumbar spinal segment and/or the first two sacral segments of the spinal cord of said mammal, especially at level L5-S1 of the spinal cord, to stimulate autonomic neurons responsible for detrusor contraction.
3. The system according to aspect 1 or aspect 2, characterized in that the at least one control unit (12) is configured and arranged to control the at least one stimulation unit (14) to provide high-frequency electrical stimulation at sacral level, especially to the first three sacral segments of the spinal cord of said mammal, to modulate sacral motoneurons responsible for external urethral sphincter relaxation,
   preferably wherein the low-frequency electrical stimulation to the last lumbar spinal segment and/or the first two sacral segments of the spinal cord of said mammal, especially at level L5-S1 of the spinal cord, and the high-frequency electrical stimulation at sacral level, especially to the first three sacral segments of the spinal cord of said mammal, are provided simultaneously.
4. The neuromodulation/neurostimulation system (10) according to any one of the preceding aspects, characterized in that the at least one control unit (12) is configured and arranged to control the at least one stimulation unit (14) to provide electrical stimulation over a predefined period of time.
5. The neuromodulation/neurostimulation system (10) according to aspect 4, characterized in that the predefined period of time is between seconds and days.
6. The neuromodulation/neurostimulation system (10) according to any one of the preceding aspects characterized in that it further comprises a sensor unit (16),
   wherein the sensor unit (16) is configured and arranged to:
   detect one or more physiological values of said mammal among:
      intra-bladder pressure;
      detrusor contraction, and/or
      external urethral sphincter pressure, and
   transmit the one or more detected physiological values to the control unit (12), and
   wherein the control unit (12) is configured and arranged to operate the stimulation unit (14) based on the one or more detected physiological values from the sensor unit (16).
7. The neuromodulation/neurostimulation system (10) according to aspect 6, characterized in that the sensor unit (16) includes one or more biocompatible implantable sensors.
8. The neuromodulation/neurostimulation system (10) according to any one of the preceding aspects characterized in that the stimulation unit (14) includes one or more implantable electrode arrays.
9. The neuromodulation/neurostimulation system (10) according to any one of the preceding aspects characterized in that the low-frequency electrical stimulation for detrusor contraction is delivered at a frequency between 10 and 700 Hz, preferably between 10 and 500 Hz, more preferably between 10 and 200 Hz,
   preferably wherein stimulation is delivered with amplitude between 0 and 50 mA, and
   preferably wherein stimulation is delivered with pulse width between 1 and 50 µs.
10. The neuromodulation/neurostimulation system (10) according to aspect 9, characterized in that the low-frequency electrical stimulation for detrusor contraction is delivered at a frequency between 10 and 500 Hz, preferably between 10 and 200 Hz, with amplitude between 0 and 50 mA and pulse width between 1 and 50 µs.
11. The neuromodulation/neurostimulation system (10) according to any one of aspects 1 to 8 characterized in that the low-frequency electrical stimulation for detrusor contraction is
   delivered according to one or more burst stimulation patterns, preferably wherein the one or more burst stimulation patterns include 0 to 20 pulses per burst.
12. The neuromodulation/neurostimulation system (10) according to aspect. 11, characterized in that the low-frequency electrical stimulation for detrusor contraction is delivered with carrier frequency between 10 and 700Hz, preferably between 10 and 500 Hz, more preferably between 10 and 200 Hz,
   preferably wherein stimulation is delivered with burst frequency between 100 and 5000 Hz, and
   preferably wherein stimulation is delivered with amplitude between 0 and 50 mA.
13. The neuromodulation/neurostimulation system (10) according to aspect. 12, characterized in that the low-frequency electrical stimulation for detrusor contraction is delivered with carrier frequency between 10 and 500 Hz, preferably between 10 and 200 Hz, with burst frequency between 100 and 5000 Hz and amplitude between 0 and 50 mA.
14. The neuromodulation/neurostimulation system (10) according to any one of the preceding aspects characterized in that the high-frequency electrical stimulation for external urethral sphincter relaxation is delivered at a frequency between 100 and 20000 Hz, preferably at 10000 Hz.
15. The neuromodulation/neurostimulation system (10) according to aspect. 14, characterized in that the high-frequency electrical stimulation for external urethral sphincter relaxation is delivered according to one or more burst stimulation patterns,
   preferably wherein said one or more burst stimulation patterns include 0 to 20 pulses per burst,
   preferably wherein stimulation is delivered with pulse width between 1 and 500 µs, and
   preferably wherein stimulation is delivered with amplitude between 0 and 50 mA.
16. The neuromodulation/neurostimulation system (10) according to any one of the preceding aspects characterized in that said mammal is a human affected by spinal cord injury (SCI) and/or other neurodegenerative or neurological disorders such as a stroke, multiple sclerosis, autonomic failure, autonomic neuropathy, and/or other neurodegenerative disorders and/or cancer of neurological tissue.
17. Use of the neuromodulation/neurostimulation system (10) according to any one of aspects 1 to 16 for the treatment of a bladder disfunction in a mammal, preferably a human.

### References

- 10, 110, 210, 310, 410, 510, 610: Neuromodulation/neurostimulation system
- 12: Control unit
- 14: Stimulation unit
- 16: Sensor unit
- 118, 218, 318, 418, 518, 618: Spinal stimulation electrodes
- 120, 220, 320, 420, 520, 620: Implanted current generator
- 222, 322, 522, 622: Controller
- 224, 524: Control sensor(s)
- 226, 326, 526, 626: Communicator
- 328, 628: Bladder sensor(s)
- 630: Urethral spincter sensor(s)
- B: Bladder
- LS: Lumbosal spinal cord
- D: Dorsal side
- US: Urethal sphincter

## Claims

1. A neuromodulation/neurostimulation system (10) for stimulating at least one neuronal circuitry responsible for micturition control in a mammal with bladder disfunction, especially for bladder relaxation and/or for bladder voiding, said system (10) comprising:
- at least one control unit (12), and
- at least one stimulation unit (14), configured and arranged to provide electrical stimulation to the spinal cord of said mammal.

2. The system according to claim 1, **characterized in that** the at least one control unit (12) is configured and arranged to control the at least one stimulation unit (14) to provide low-frequency electrical stimulation to the last lumbar spinal segment and/or the first two sacral segments of the spinal cord of said mammal, especially at level L5-S1 of the spinal cord, to stimulate autonomic neurons responsible for detrusor contraction.

3. The system according to claim 1 or claim 2, **characterized in that** the at least one control unit (12) is configured and arranged to control the at least one stimulation unit (14) to provide high-frequency electrical stimulation at sacral level, especially to the first three sacral segments of the spinal cord of said mammal, to modulate sacral motoneurons responsible for external urethral sphincter relaxation,
preferably wherein the low-frequency electrical stimulation to the last lumbar spinal segment and/or the first two sacral segments of the spinal cord of said mammal, especially at level L5-S1 of the spinal cord, and the high-frequency electrical stimulation at sacral level, especially to the first three sacral segments of the spinal cord of said mammal, are provided simultaneously.

4. The neuromodulation/neurostimulation system (10) according to any one of the preceding claims, **characterized in that** the at least one control unit (12) is configured and arranged to control the at least one stimulation unit (14) to provide electrical stimulation over a predefined period of time.

5. The neuromodulation/neurostimulation system (10) according to claim 4, **characterized in that** the predefined period of time is between seconds and days.

6. The neuromodulation/neurostimulation system (10) according to any one of the preceding claims, **characterized in that** it further comprises a sensor unit (16),
wherein the sensor unit (16) is configured and arranged to:
detect one or more physiological values of said mammal among:
intra-bladder pressure;
detrusor contraction, and/or
external urethral sphincter pressure, and
transmit the one or more detected physiological values to the control unit (12), and
wherein the control unit (12) is configured and arranged to operate the stimulation unit (14) based on the one or more detected physiological values from the sensor unit (16).

7. The neuromodulation/neurostimulation system (10) according to claim 6, **characterized in that** the sensor unit (16) includes one or more biocompatible implantable sensors.

8. The neuromodulation/neurostimulation system (10) according to any one of the preceding claims **characterized in that** the stimulation unit (14) includes one or more implantable electrode arrays.

9. The neuromodulation/neurostimulation system (10) according to any one of the preceding claims **characterized in that** the low-frequency electrical stimulation for detrusor contraction is delivered at a frequency between 10 and 700 Hz, preferably between 10 and 500 Hz, more preferably between 10 and 200 Hz,
preferably wherein stimulation is delivered with amplitude between 0 and 50 mA, and
preferably wherein stimulation is delivered with pulse width between 1 and 50 µs.

10. The neuromodulation/neurostimulation system (10) according to claim 9, **characterized in that** the low-frequency electrical stimulation for detrusor contraction is delivered at a frequency between 10 and 500 Hz, preferably between 10 and 200 Hz, with amplitude between 0 and 50 mA and pulse width between 1 and 50 µs.

11. The neuromodulation/neurostimulation system (10) according to any one of claims 1 to 8 **characterized in that** the low-frequency electrical stimulation for detrusor contraction is delivered according to one or more burst stimulation patterns, preferably wherein the one or more burst stimulation patterns include 0 to 20 pulses per burst.

12. The neuromodulation/neurostimulation system (10) according to claim 11, **characterized in that** the low-frequency electrical stimulation for detrusor contraction is delivered with carrier frequency between 10 and 700Hz, preferably between 10 and 500 Hz, more preferably between 10 and 200 Hz,
preferably wherein stimulation is delivered with burst frequency between 100 and 5000 Hz, and
preferably wherein stimulation is delivered with amplitude between 0 and 50 mA.

13. The neuromodulation/neurostimulation system (10) according to claim 12, **characterized in that** the low-frequency electrical stimulation for detrusor contraction is delivered with carrier frequency between 10 and 500 Hz, preferably between 10 and 200 Hz, with burst frequency between 100 and 5000 Hz and amplitude between 0 and 50 mA.

14. The neuromodulation/neurostimulation system (10) according to any one of the preceding claims **characterized in that** the high-frequency electrical stimulation for external urethral sphincter relaxation is delivered at a frequency between 100 and 20000 Hz, preferably at 10000 Hz.

15. The neuromodulation/neurostimulation system (10) according to claim 14, **characterized in that** the high-frequency electrical stimulation for external urethral sphincter relaxation is delivered according to one or more burst stimulation patterns,
preferably wherein said one or more burst stimulation patterns include 0 to 20 pulses per burst,
preferably wherein stimulation is delivered with pulse width between 1 and 500 µs, and
preferably wherein stimulation is delivered with amplitude between 0 and 50 mA.

16. The neuromodulation/neurostimulation system (10) according to any one of the preceding claims, **characterized in that** said mammal is a human affected by spinal cord injury (SCI) and/or other neurodegenerative or neurological disorders such as a stroke, multiple sclerosis, autonomic failure, autonomic neuropathy, and/or other neurodegenerative disorders and/or cancer of neurological tissue.

17. Use of the neuromodulation/neurostimulation system (10) according to any one of claims 1 to 16 for the treatment of a bladder disfunction in a mammal, preferably a human.
